# EUROPEAN PATENT SPECIFICATION

(11) **EP 2 646 017 B1**
(45) Date of publication and mention of the grant of the patent: **16.11.2016**
(21) Application number: 11794357.1
(22) Date of filing: 30.11.2011
(51) Int. Cl.: A61K 31/381, A61K 31/343, A61K 9/00, A61K 8/42, A61K 8/49, A61K 31/167, A61K 31/4015, A61K 31/421, A61K 31/519, A61P 17/02, A61Q 19/08, A61K 8/37, A61K 31/22

(54) **COMPOUNDS AND METHODS FOR SKIN REPAIR**
VERBINDUNGEN UND VERFAHREN ZUR HAUTREPARATUR
COMPOSÉS ET PROCÉDÉS DE RÉPARATION DE LA PEAU

(30) Priority: 02.12.2010 US 419115 P
(43) Date of publication of application: 09.10.2013
(73) Proprietor: ALLERGAN, INC., Irvine, CA 92612 (US)
(72) Inventor: BURK, Robert, M., Laguna Beach CA 92651 (US); IM, Wha Bin, Irvine CA 92612 (US); WHITCUP, Scott, M., Laguna Hills CA 92653 (US)
(74) Representative: Hoffmann Eitle
(86) International application number: PCT/US2011/062691
(87) International publication number: WO 2012/075174

(56) References cited:
- EP-A1- 1 114 816
- EP-A1- 1 417 975
- EP-A1- 1 563 846
- WO-A1-03/007941
- WO-A1-2006/137472
- WO-A1-2007/115001
- WO-A1-2008/071736
- WO-A1-2008/076703
- WO-A1-2008/092860
- WO-A1-2008/092861
- WO-A1-2008/092862
- WO-A1-2012/024072
- WO-A2-2009/150118
- WO-A2-2012/016109
- US-A- 5 877 211
- US-A1- 2004 142 969
- US-A1- 2005 065 133
- US-A1- 2011 111 031
- CHUN KYUNG-SOO ET AL: "Cyclooxygenase-2 inhibits UVB-induced apoptosis in mouse skin by activating the prostaglandin E-2 receptors, EP2 and EP4", CANCER RESEARCH, vol. 67, no. 5, March 2007 (2007-03), pages 2015-2021, XP002662646, ISSN: 0008-5472
- SAKAI Y ET AL: "Prostaglandin E2 regulates the expression of basic fibroblast growth factor messenger RNA in normal Human Fibroblasts", KOBE JOURNAL OF MEDICAL SCIENCES, vol. 47, no. 35-45, 1 February 2001 (2001-02-01), pages 35-45, XP002974917, KOBE UNIVERSITY SCHOOL OF MEDICINE, KOBE, JP ISSN: 0023-2513
- LI Y-J ET AL: "Prostaglandin E2 inhibits human lung fibroblast chemotaxis through disparate actions on different E-prostanoid receptors", AMERICAN JOURNAL OF RESPIRATORY CELL AND MOLECULAR BIOLOGY, vol. 44, no. 1, 1 January 2010 (2010-01-01), pages 99-107, XP009153618, AMERICAN LUNG ASSOCIATION, NEW YORK, NY, US ISSN: 1044-1549, DOI: 10.1165/RCMB.2009-0163OC
- DATABASE PubChem Compound [Online] 25 October 2006 (2006-10-25), "ONO-AE1-329 - Compound Summary", XP002674561, retrieved from NCBI Database accession no. 9846782

## Description

### FIELD OF THE INVENTION

The invention relates to EP4 agonists for usein the treatment and prevention of scars.

### BACKGROUND OF THE INVENTION

Prostanoid EP4 receptor is a G protein-coupled receptor that mediates the actions of prostaglandin E2 (PGE2) and is characterized by the longest intracellular C terminus loop when compared to other prostanoid receptors. EP4 receptors couple not only to Gs and mediate elevations in cAMP concentration, but also to Gi and phosphorylate key intracellular signaling proteins such as ERK or AKT . EP2 is another PGE2 receptor subtype analogous to EP4. There are some redundancies in function between EP2 and EP4 receptors. For example, both receptors induce IOP lowering, and are involved in PGE2-mediated RANKL through cAMP signalings. There are some functional differences, however, primarily arising from differences in receptor density at the target sites: Thus, EP2 is involved in cumulus expansion in ovulation and fertilization, and EP4 regulates closure of the ductus arteriosus. Expression of EP4 receptors is controlled by various physiological and pathophysiological processes as these receptors participate in ovulation and fertilization, induce bone formation, protect against inflammatory bowel disease, facilitate Langerhans cell migration and maturation and mediate joint inflammation in a model of collagen-induced arthritis, among others

Skin blemishes such as flesh wounds, scars from cosmetic surgery including, but not limited to, breast implants, from surgeries on the back, central chest, heart, abdomen, pubic area, and joint, from burns, aging, and photoaging, and wrinkles can occur on any area of the body. Scarring may occur in all parts of adult body, following local or systemic traumas such as mechanical injury, surgery, burn, chemical contact, radiation and poisoning, and represents a failure of homeostatic processes to restore normal structure at the wound sites. Wrinkles occur for a variety of reasons and are a common sign of aging. Both scars and signs of aging can typically considered undesirable. Moreover, the prevention of unsightly scars from any injuries or in people at high risk of bad scars is highly desirable.

Accordingly, an agent that safely and effectively treats or prevents scars is highly desirable.

### SUMMARY OF THE INVENTION

The disclosure provides compositions for use in treating or preventing scars. Scars that can be treated or prevented by the compositions of the invention can arise from events such as surgery on all parts of the body, trauma, disease, mechanical injury, burn, radiation, poisoning, photoaging, aging, chemical contact and the like.

### BRIEF DESCRIPTION OF THE DRAWINGS

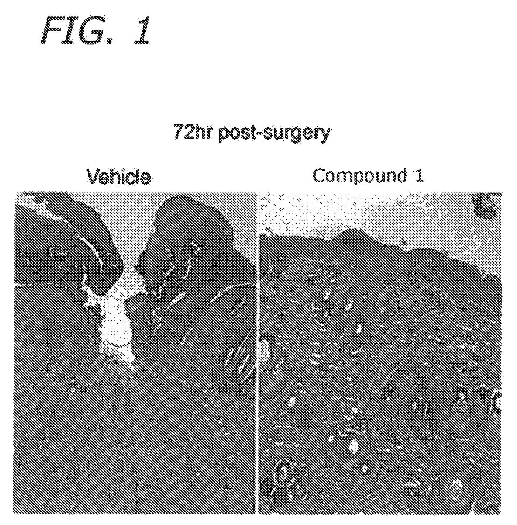

**Figure 1** is an image of a hematoxylin & eosin (H&E) stained skin biopsy samples at 3 days post skin incisional surgery. The image shows epidermal coverage of the wound site (magnification 200x).

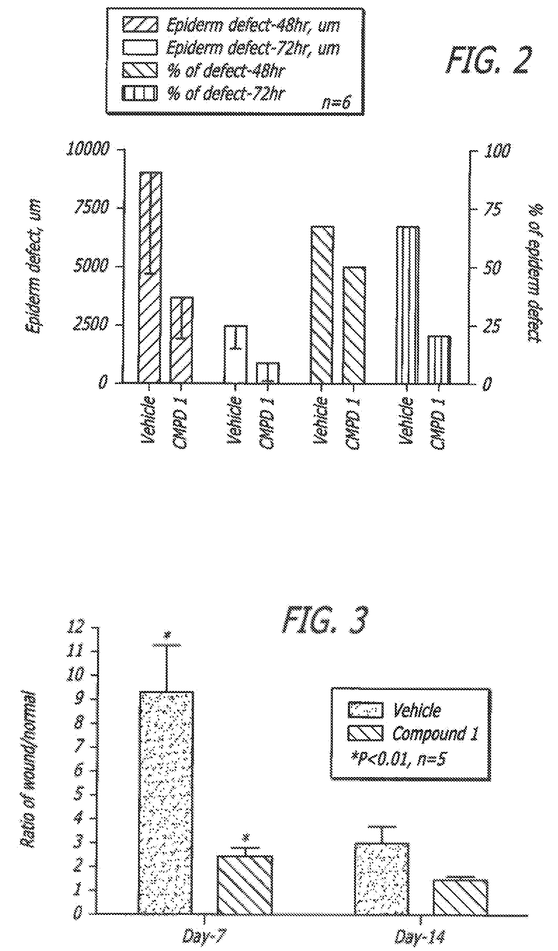

**Figure 2** is a graph showing an epidermal defect (µm and percentage) at 2 and 3 days post-surgery for vehicle treated and the non-claimed EP4 agonist "Compound 1" treated groups. 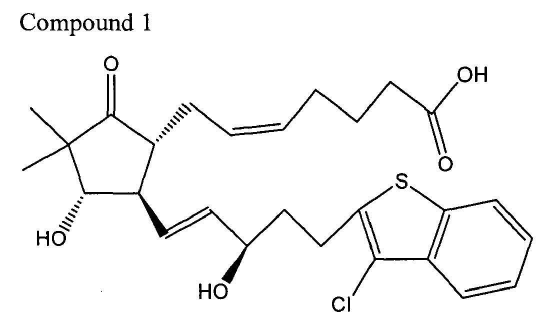
**Figure 3** is a graph showing epidermal thickness at wound sites compared to nearby normal sites (ratio wound/normal) at 7 and 14 days post-surgery in groups treated with vehicle and Compound 1.

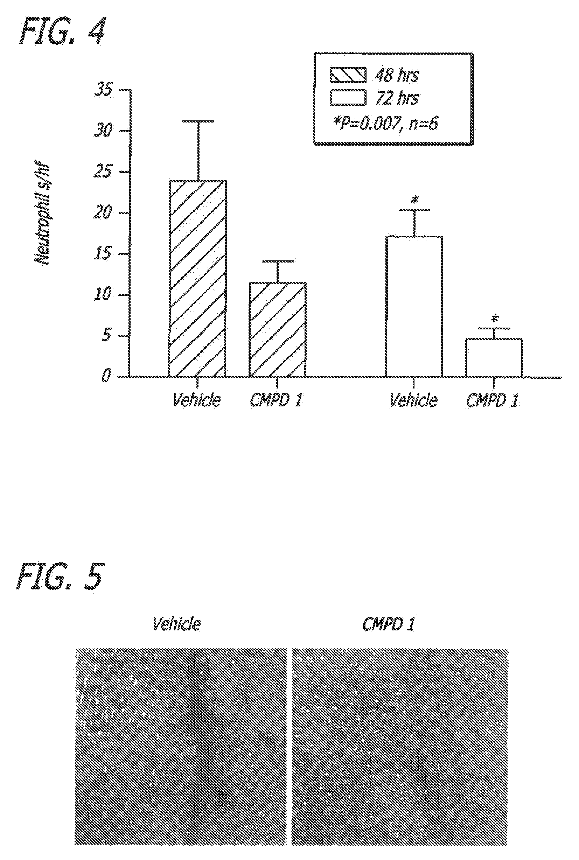

**Figure 4** is a graph showing quantification of neutrophils (s/hf) at wound sites at 2 and 3 days post-surgery in groups treated with vehicle and Compound 1. Neutrophils at the dermis region were counted under 400x magnification.
**Figure 5** is an image showing macroscopic appearances of skin wound sites at 14 days post-surgery in vehicle treated and Compound 1 treated skin at a magnification of 6.5x.

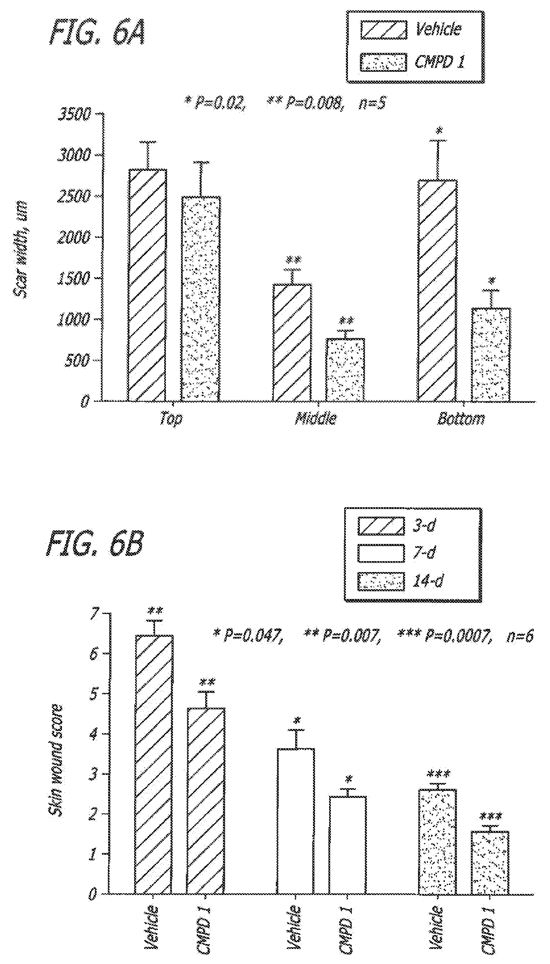

**Figures 6**A and **B** are graphs quantifying skin scar tissue sections and gross tissue appearance of samples treated with either vehicle or Compound 1. **Figure 6A** shows scar width (µm) on Masson trichrome stained sections at the top, middle and bottom of the section. **Figure 6B** shows the gross skin wound score at days 3, 7, and 14 post-surgery.

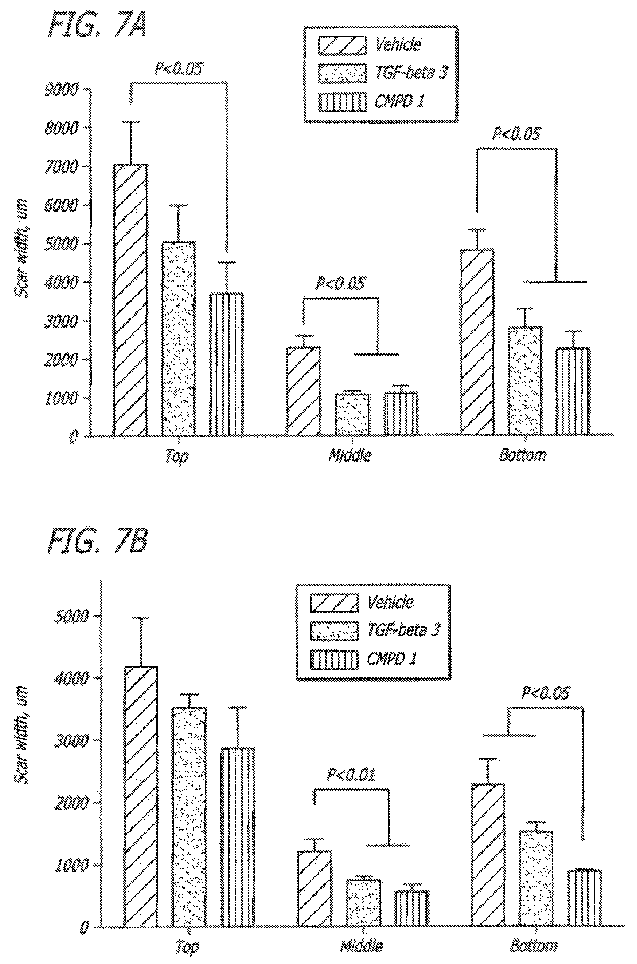

**Figures 7A** and **B** are graphs quantifying skin scar width on wound sections at 2 weeks post-surgery. **Figure 7A** shows scar width (µm) of picrosirius red stained sections in the top, middle and bottom. **Figure 7B** shows scar width at the top, middle and bottom sections of Masson trichrome stained sections treated with either vehicle, TGF-β3, or Compound 1.

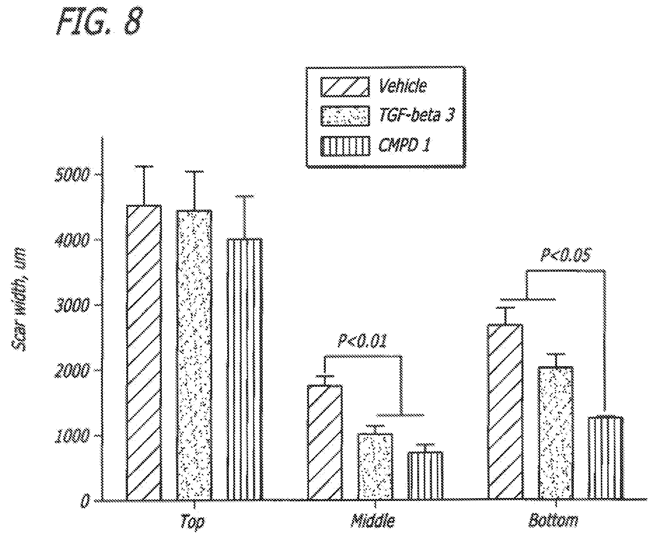

**Figure 8** is a graph quantifying skin scar width based on Masson trichrome staining 70 days post-surgery in tissue treated with vehicle, TGF-β3, or Compound 1.

### DETAILED DESCRIPTION OF THE INVENTION

It is to be understood that both the foregoing general description and the following detailed description are exemplary and explanatory only and are not restrictive of the invention claimed. As used herein, the use of the singular includes the plural unless specifically stated otherwise. As used herein, "or" means "and/or" unless stated otherwise. Furthermore, use of the term "including" as well as other forms, such as "includes," and "included," is not limiting. The section headings used herein are for organizational purposes only and are not to be construed as limiting the subject matter described.

Unless specific definitions are provided, the nomenclatures utilized in connection with, and the laboratory procedures and techniques of analytical chemistry, synthetic organic and inorganic chemistry described herein are those known in the art. Standard chemical symbols are used interchangeably with the full names represented by such symbols. Thus, for example, the terms "hydrogen" and "H" are understood to have identical meaning. Standard techniques may be used for chemical syntheses, chemical analyses, and formulation.

Disclosed herein are compositions for scar reduction.

In one embodiment of the invention, a composition is provided for use in treating or preventing scars, the composition comprising a therapeutically effective amount of at least one compound having a structure:

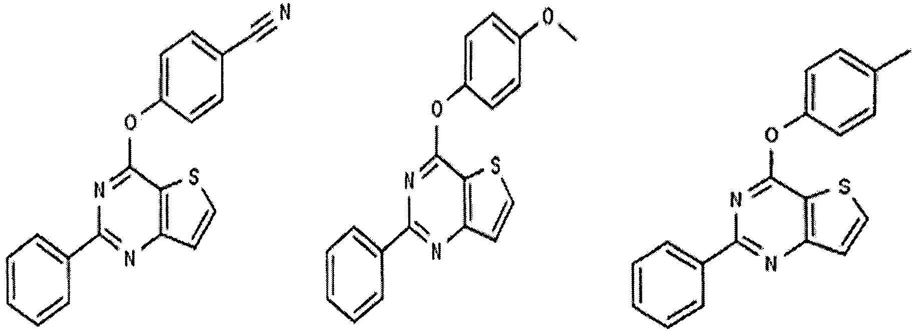

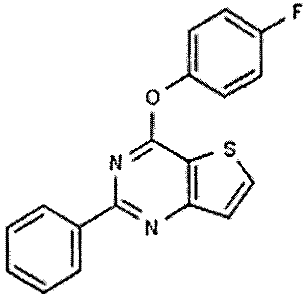

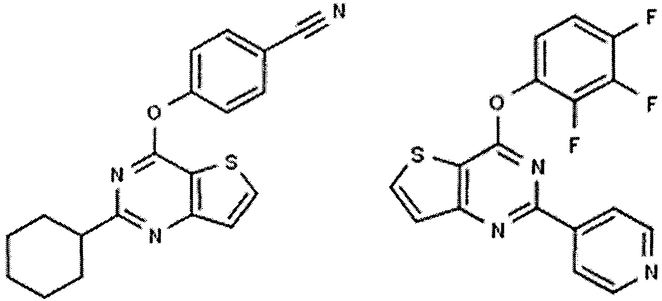

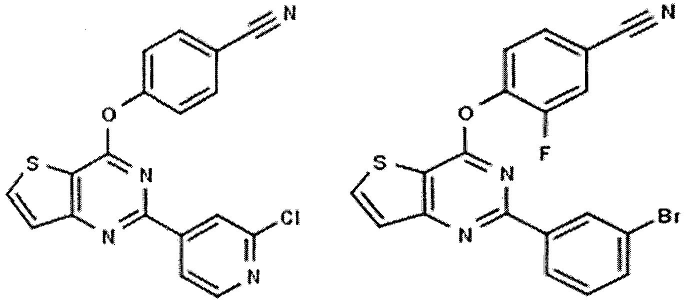

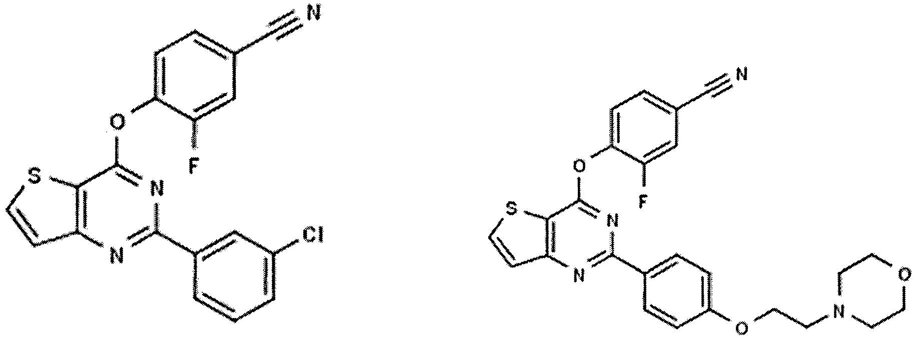

Methods of preparing the disclosed compounds suitable for use in the methods disclosed herein, can be found in, e.g., WO 2008/092860 A1, WO 2008/092861 A1 and WO 2008/092862 A1.

A "scar" is an area of abnormal skin appearances arising from fibrous tissue formation (fibrosis) or sclerosis, for example scleroderma, or a loss of normal skin components , after various surgical procedures, injury, burn, irradiation, chemical contact, or diseases including various infections on all parts of the body. Scar types include, but not limited to, hypertrophic scars, recessed scars, and stretch marks. Hypertrophic scars occur when the body overproduces collagen, which causes the scar to be raised above the surrounding skin. An example of a hypertrophic scar is a keloid scar, including the prevention of recurrence of fibrous tissue growth after excision of existing keloid scars. Atrophic, or recessed scars, have a sunken appearance and result when underlying support structure in the skin is lost. Stretch marks (striae) occur when skin is stretched rapidly (i.e., due to significant weight gain or growth spurt or post pregnancy), or when skin is put under tension during the healing process, typically near a joint. As used herein, the term "scar" encompasses any type of scar in the skin due to any cause.

As used herein, "treatment" means to prevent and alleviate (or to eliminate) scars either temporarily or permanently. When the compositions are administered to treat a wound, the compositions promote normal healing compared to a wound without the administration. That is, the size (length, depth, height and/or width), character, color and/or texture of the treated wound more closely resemble normal, non-wounded tissue. In this regard, treatment of a wound with the disclosed compositions can prevent, minimize or improve the appearance of a scar formation resulting from healing of the wound.

The disclosed compositions can be administered to prevent scar formation not associated with a wound, such as a stretch mark, or scars resulting from acne, chicken pox, measles or other disease states. In certain embodiments, the disclosed compositions are administered to the area of skin expansion in order to prevent formation of such scars. In these embodiments, the composition can be administered to any region of a face, abdomen, breasts, arms, legs, buttocks, back, or any other area where the skin is susceptible to developing a scar.

The compositions can be administered prior to, concurrently with, and/or after the development of the skin blemish. For instance, the disclosed compositions can be administered prior to an incision, during a surgical procedure, and/or any time postoperatively, and then additionally administered after the procedure as the healing process occurs. In another example, the compositions can be administered during pregnancy to prevent stretch marks. Alternately, the compositions can be administered after the development of a blemish.

The compositions may be administered typically for 1 to 7 days , or for a period of time necessary to achieve the desired results, which may be several days to several months. The compositions can be administered once or several times (2, 3, 4, or more times) a day depending on the desired effect. In certain embodiments, the compositions can be administered every 1, 2, 3, 4, 5, 6, or 7 days. In another embodiment, the compositions can be administered one or more times every 1, 2, 3, or 4 weeks. The administration can be on a monthly or bi-monthly basis. Further, the compositions can be administered for 1, 2, 3, 6, 9, or 12 months or more. In certain embodiments, the compositions can be administered on an ongoing basis to maintain a desired result.

The disclosed compounds can be administered as part of a composition. As used herein, "formulation" and "composition" may be used interchangeably and refer to a combination of elements that is presented together for a given purpose. Such terms are well known to those of ordinary skill in the art.

As used herein, "carrier," "inert carrier," and "acceptable carrier" may be used interchangeably and refer to a carrier which may be combined with the presently disclosed compounds in order to provide a desired composition. Those of ordinary skill in the art will recognize a number of carriers that are well known for making specific pharmaceutical and/or cosmetic compositions. Desirably, the carrier is suitable for application to keratinous surfaces or other areas of the body. Upon application, acceptable carriers are substantially free of adverse reactions with skin and other keratinous surfaces. For example, the carriers may take the form of fatty or non-fatty creams, milky suspensions or emulsion-in-oil or oil-in-water types, lotions, gels or jellies, colloidal or non-colloidal aqueous or oily solutions, pastes, aerosols, soluble tablets or sticks. In accordance with one embodiment, the composition includes a dermatologically compatible vehicle or carrier. The vehicle which may be employed for preparing compositions may comprise, for example, aqueous solutions such as e.g., physiological salines, oil solutions or ointments. The vehicle furthermore may contain dermatologically compatible preservatives such as e.g., benzalkonium chloride, surfactants like e.g., polysorbate 80, liposomes or polymers, for example, methyl cellulose, polyvinyl alcohol, polyvinyl pyrrolidone and hyaluronic acid; these may be used for increasing the viscosity.

Examples of additional agents which can be included in the present compositions are anti-itch, anti-cellulite, anti-scarring, and anti-inflammatory agents, anesthetics, anti-irritants, vasoconstrictors, vasodilators, as well as agents to prevent/stop bleeding, and improve/remove pigmentation, moisturizers, desquamating agents, tensioning agents, anti-acne agents. Anti-itch agents can include methyl sulphonyl methane, sodium bicarbonate, calamine, allantoin, kaolin, peppermint, tea tree oil and combinations thereof. Anti-cellulite agents can include forskolin, xanthine compounds such as, but not limited to, caffeine, theophylline, theobromine, and aminophylline, and combinations thereof. Anesthetic agents can include lidocaine, benzocaine, butamben, dibucaine, oxybuprocaine, pramoxine, proparacaine, proxymetacaine, tetracaine, and combinations thereof. Anti-scarring agents can include IFN-.gamma., fluorouracil, poly(lactic-co-glycolic acid), methylated polyethylene glycol, polylactic acid, polyethylene glycol and combinations thereof. Anti-inflammatory agents can include dexamethasone, prednisolone, corticosterone, budesonide, estrogen, sulfasalazine, mesalamine and derivatives and combinations thereof. Additionally, active agents such as epinephrine, thymidine, cytidine, uridine, antiypyrin, aminocaproic acid, tranexamic acid, eucalyptol, allantoin, glycerin, and sodium selenite, can be included. Formulations can further comprise degradation inhibitors. Degradation inhibitors, include but are not limited to, glycosaminoglycans (e.g., heparin, heparin sulfate, dermatan sulfate, chrondroitin sulfate, o-sulfated HA, lnamarin, and amygdalin), antioxidants (e.g. ascorbic acid, melatonin, vitamin C, vitamin E), proteins (e.g., serum hyaluronidase inhibitor), and fatty acids (e.g. saturated C₁₀ to C₂₂ fatty acids). In certain embodiments, additional active agent is an antioxidant. In certain embodiments, the antioxidant comprises a vitamin C and/or a vitamin E such as d-alpha-tocopheryl polyethylene glycol 1000 succinate (TPGS).

The disclosed compositions are well suited for topical, subcutaneous, intradermal, subdermal, subcutaneous, and transdermal administration. Topical administration relates to the use of a composition applied to the surface of the skin at the site of a skin blemish for exertion of local action. Accordingly, such topical compositions include those pharmaceutical or cosmetic forms in which the composition is applied externally by direct contact with the skin surface to be treated, such as the face, neck, arms, legs, and/or torso. Conventional pharmaceutical or cosmetic forms for this purpose include ointments, liniments, creams, shampoos, lotions, pastes, jellies, sprays, aerosols, and the like, and may further be applied directly or in patches or impregnated dressings depending on blemish and skin region to be treated. The term "ointment" embraces formulations (including creams) having oleaginous, water-soluble and emulsion-type bases, e.g., petrolatum, lanolin, polyethylene glycols, as well as mixtures of these.

The compositions are appropriate for mesotherapy applications as well. Mesotherapy is a non-surgical cosmetic treatment technique involving intra-epidermal, intra-dermal, and/or subcutaneous injection of a composition. The compositions are administered in the form of small multiple droplets into the epidermis, dermo-epidermal junction, and/or the dermis.

In accordance with the disclosure, a pharmaceutical or cosmetic composition can optionally include one or more agents such as, without limitation, emulsifying agents, wetting agents, sweetening or flavoring agents, tonicity adjusters, preservatives, buffers antioxidants and flavonoids. Tonicity adjusters useful in a pharmaceutical composition of the present disclosure include, but are not limited to, salts such as sodium acetate, sodium chloride, potassium chloride, mannitol or glycerin and other pharmaceutically acceptable tonicity adjusters. Preservatives useful in the pharmaceutical compositions described herein include, without limitation, benzalkonium chloride, chlorobutanol, thimerosal, phenyl mercuric acetate, and phenyl mercuric nitrate. Various buffers and means for adjusting pH can be used to prepare a pharmaceutical composition, including but not limited to, acetate buffers, citrate buffers, phosphate buffers and borate buffers. Similarly, antioxidants useful in pharmaceutical compositions are well known in the art and include for example, sodium metabisulfite, sodium thiosulfate, acetylcysteine, butylated hydroxyanisole and butylated hydroxytoluene. Flavonoids are compounds found in plants that are well known to have diverse beneficial biochemical and antioxidant effects. Subcategories of flavonoids include: flavones, flavonols, flavanonse and flavanonols. Examples of flavonoids include: luteolin, apigenin, tangeritin, quercetin, kaempferol, myricetin, fisetin, isorhamnetin, pachypodol, rhamnazin, hesperetin, naringenin, eriodictyol, homoeriodictyol, taxifolin, dihydroquercetin, dihydrokaempferol, tannic acid, tannis, condensed tannis, and hydrolysable tannis. It is understood that these and other substances known in the art can be included in a pharmaceutical or cosmetic composition disclosed herein.

As used herein, the term "therapeutically effective amount" means the amount of the pharmaceutical or cosmetic composition that will elicit the biological, medical, or cosmetic response of a subject in need thereof that is being sought by the researcher, veterinarian, medical doctor or other clinician. In some embodiments, the subject in need thereof is a mammal. In certain embodiments, the mammal is human. Effective amounts of the compound may be determined by one of ordinary skill in the art but will vary depending on the compound employed, frequency of application and desired result, and will generally range from about 0.0000001% to about 50%, by weight, of the composition, preferably from about 0.001% to about 50%, by weight, of total composition, more preferably from about 0.001% to about 30%, by weight of the composition. In certain embodiments, the compound is about 0.004% by weight of the composition.

The compounds described herein may be administered at least in the minimum dose necessary to achieve the desired therapeutic effect. Generally, such doses will be in the range of about 1 mg/day to about 1000 mg/day; more preferably in the range of about 10 mg/day to about 500 mg/day. In another example embodiment, the compound or compounds may be present in a composition or formulation in a range of about 0.0001 mg/kg/day to about 100 mg/kg/day or about 0.01 mg/kg/day to about 100 mg/kg/day. However, the actual amount of the compound to be administered in any given case will be determined by a physician taking into account the relevant circumstances, such as the age and weight of a patient, patient's general physical condition, severity of the skin blemish, and route of administration. In some instances, dosing is evaluated on a case-by-case basis.

Additionally, compositions may be designed to delay release of the compound over a given period of time, or to carefully control the amount of compound released at a given time during the course of treatment.

The pH of the disclosed compositions can be about 3 to about 8.0, or about 6.5 to about 7.5. In certain embodiments, the pH of the formulation is about 7.0 to about 7.4 or about 7.1 to about 7.3.

Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

## Claims

1. A composition for use in the treatment and prevention of scars comprising a therapeutically effective amount of at least one EP4 agonist having the structure:

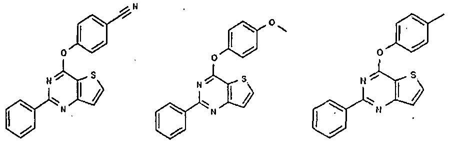

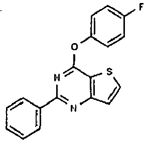

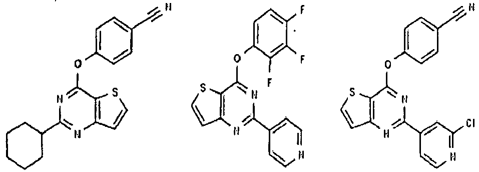

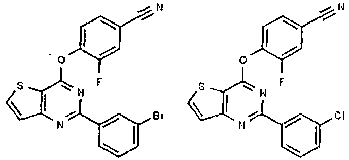

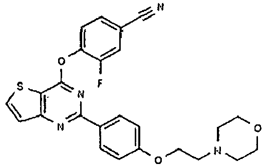

2. The composition for use according to claim 1 which is for topical, intradermal, subdermal, subcutaneous and transdermal administration.

## Patentansprüche

1. Zusammensetzung zur Verwendung bei der Behandlung und Prävention von Narben, umfassend eine therapeutisch wirksame Menge von zumindest einem EP4-Agonisten mit der Struktur:

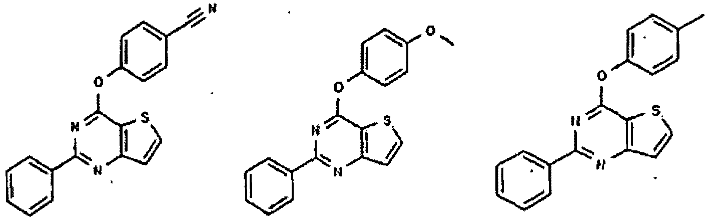

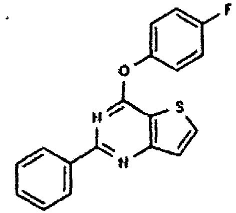

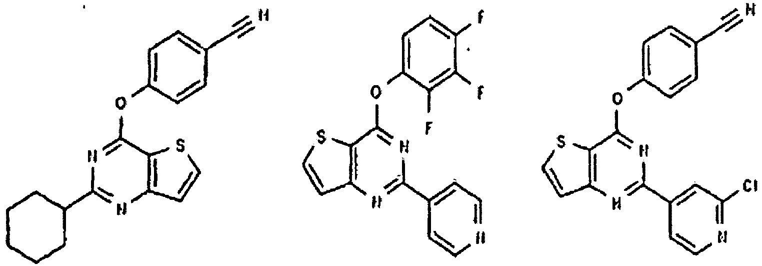

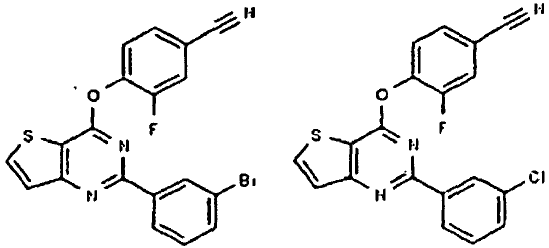

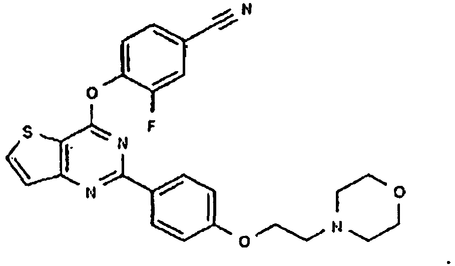

2. Zusammensetzung zur Verwendung gemäss Anspruch 1, die zur topischen, intradermalen, subdermalen, subkutanen und transdermalen Verabreichung dient.

## Revendications

1. Composition à utiliser dans le traitement et la prévention de cicatrices, comprenant une quantité thérapeutiquement efficace d'au moins un agoniste EP4 présentant la structure :

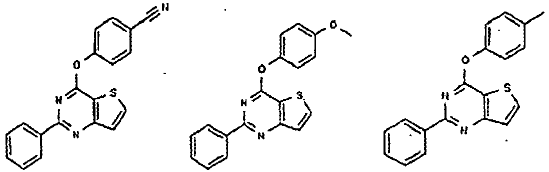

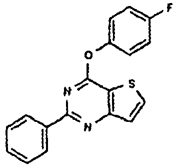

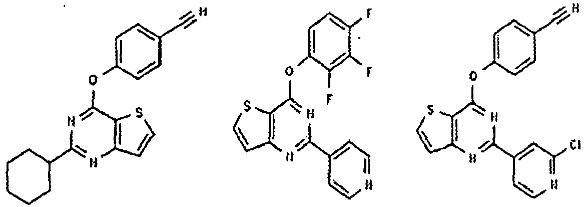

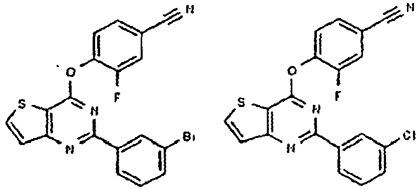

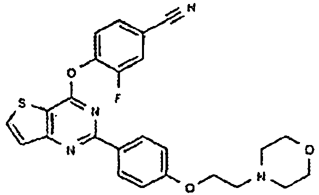

2. Composition à utiliser selon la revendication 1, qui est adaptée à une administration topique, intradermique, sous-dermique, sous-cutanée et transdermique.
